# EUROPEAN PATENT APPLICATION

(11) **EP 4 674 291 A1**
(43) Date of publication of application: **07.01.2026**
(21) Application number: 24187743.0
(22) Date of filing: 10.07.2024
(51) Int. Cl.: A24F 40/46

(54) **AEROSOL GENERATORS AND DELIVERY SYSTEMS**

(30) Priority: 01.07.2024 CN 202410879208
(71) Applicant: Nicoventures Trading Limited, London WC2R 3LA (GB)
(72) Inventor: ZHAO, Cihai, London, WC2R 3LA (GB); LIN, Shengyang, London, WC2R 3LA (GB); ZHOU, Yukun, London, WC2R 3LA (GB)
(74) Representative: D Young & Co LLP

(57) **Abstract**

An aerosol generator 48 for an aerosol delivery system 1, the aerosol generator 48 having an electrically conductive portion 100 for generating aerosol from aerosol-generating material in use, wherein the conductive portion 100 extends from a first surface 71 of the aerosol generator 48 to a second, different surface 72 of the aerosol generator 48.

## Description

### Field

This disclosure relates to aerosol generators for aerosol delivery systems, which may include nicotine delivery systems, as well as aerosol delivery systems themselves.

### Background

Aerosol delivery systems such as electronic cigarettes (e-cigarettes) generally contain an aerosol generating material, such as a chamber of a source solid or liquid, which may contain an active substance and / or a flavour, from which an aerosol or vapour is generated for inhalation by a user, e.g. through heat vaporisation. An aerosol delivery system typically comprises an aerosol generation area containing an aerosol generator, e.g. a heating element, arranged to vaporise or aerosolise a portion of precursor material to generate a vapour or aerosol in the aerosol generation area. As a user inhales on the system and electrical power is supplied to the vaporiser, air is drawn into the system through an inlet hole and along an inlet air channel connecting to the aerosol generation area, where the air mixes with vaporised precursor material to form a condensation aerosol. There is an outlet channel connecting the aerosol generation area to an outlet in a mouthpiece and the air drawn into the aerosol generation area as a user inhales on the mouthpiece continues along the outlet flow path to the mouthpiece outlet, carrying the aerosol with it, for inhalation by the user.

Some electronic cigarettes may include a flavour element in the air flow path to impart additional flavours. Such systems may be referred to as hybrid devices, and the flavour element may, for example, include a portion of tobacco arranged in the air flow path between the aerosol generation area and the mouthpiece such that vapour / aerosol drawn through the device passes through the portion of tobacco before exiting the mouthpiece for user inhalation.

It is of interest to develop aerosol generators and aerosol delivery systems delivering larger droplets of vapour in aerosol delivered to the user, for improved sensory experience. Various approaches are described herein which seek to help address or mitigate at least some of the issues discussed above.

### Brief summary of the invention

In one aspect, there is provided an aerosol generator for an aerosol delivery system, the aerosol generator having an electrically conductive portion for generating aerosol from aerosol-generating material in use, wherein the conductive portion extends from a first surface of the aerosol generator to a second, different surface of the aerosol generator.

In another aspect, there is provided an aerosol generator for an aerosol delivery system, the aerosol generator having an electrically conductive portion for generating aerosol from aerosol-generating material in use, wherein the conductive portion comprises a curved surface having a curvature angle of substantially 30-150°, 45-135° or 45-90°.

In another aspect, there is provided an aerosol delivery system or a cartridge for an aerosol delivery system, comprising: an aerosol generator having an electrically conductive portion for generating aerosol from aerosol-generating material in use; an electrically conductive element for connecting the electrically conductive portion to a power supply; and a flow path past the conductive portion for entraining aerosol generated by the aerosol generator into air from an air inlet, wherein the conductive portion is exposed to the flow path and the conductive element does not protrude beyond the conductive portion towards the flow path.

The claimed invention further provides corresponding functional means and further provides additional embodiments as claimed in the dependent claims.

In some examples, the first surface and/or the second surface is substantially planar.

In some examples, the first and second surfaces are adjacent to one another. In some examples, the first and second surfaces are not adjacent to one another.

In some examples, the first and second surfaces are spaced from one another. In some examples, the first and second surfaces are spaced from one another by a third, different surface. In some examples, the first and second surfaces are substantially parallel to one another. In some examples, the first and second surfaces are not parallel to one another.

In some examples, an angle between the first surface and the second surface is substantially 30-150°, 45-135° or 45-90°.

In some examples, an angle between the conductive portion of the first surface and the conductive portion of the second surface is substantially 30-150°, 45-135° or 45-90°.

In some examples, the conductive portion comprises an 'L'-shape.

In some examples, the aerosol generator has an electrically conductive portion for generating aerosol from aerosol-generating material by resistance heating in use.

In some examples, the first and second surfaces are substantially perpendicular.

In some examples, the conductive portion comprises a curved surface having a curvature angle of substantially 90°, which effectively provides the same arrangement as two perpendicular planar surfaces, where tangents at each end of a substantially 90° curved surface are substantially perpendicular.

In some examples, the first surface comprises a vaporisation surface or portion. In some examples, the vaporisation surface or portion is configured to receive aerosol-generating material and generate vapour/aerosol therefrom, optionally by resistance heating. In some examples, the second surface comprises a power supply surface or portion configured to receive power from a power supply. In some examples, the power supply surface or portion does not receive aerosol-generating material. In some examples, the power supply surface is substantially impermeable.

In some examples, the conductive portion comprises a first, substantially planar conductive portion on the first surface, connected to a second, substantially planar conductive portion on the second surface.

In some examples, the conductive portion extends from the first surface of the aerosol generator to multiple other surfaces of the aerosol generator, which may each be substantially perpendicular to the first surface.

In some examples, the conductive portion extends externally across and/or internally between surfaces of the aerosol generator.

Some examples comprise an aerosol delivery system or a cartridge for an aerosol delivery system, the system or cartridge comprising the aerosol generator of any example, optionally further comprising aerosol-generating material.

In some examples, the aerosol generator comprises a porous body, configured to transport aerosol-generating material to the conductive portion for aerosolisation.

In some examples, the aerosol generator comprises a substantially rectangular body.

In some examples, the aerosol generator comprises a porous body and/or a ceramic body, which may be suitable for retaining and/or transporting aerosol-generating material to the conductive portion.

In some examples, the conductive portion comprises a trace, thin-film or etched conductive portion.

In some examples, the aerosol generator, system or cartridge comprising a reservoir of aerosol-generating material configured to deliver aerosol-generating material to a surface of the aerosol generator that substantially opposes the first or the second surface of the aerosol generator.

In some examples, the reservoir of aerosol-generating material is configured to deliver aerosol-generating material to a surface of the aerosol generator opposing the vaporisation surface. The porous aerosol generator body may transport aerosol-generating material from the reservoir to the conductive portion.

In some examples, when the conductive portion comprises a vaporisation surface that is orientated to have a depth extending horizontally, the reservoir of aerosol-generating material is vertically above the aerosol generator and configured to deliver aerosol-generating material under the influence of gravity (in this orientation) to a top surface of the aerosol generator.

In some examples, the conductive portion comprises: a higher resistance portion (or part); and/or a lower resistance portion (or part).

In some examples, the conductive portion comprises:
a. a higher resistance portion, for generating aerosol from aerosol-generating material by resistance heating; and
b. a lower resistance portion, for supplying power to the higher resistance portion.

In some examples, the higher resistance portion has a resistance that is at least 2, 3, 4, 5, 6, 7, 8, 9 or 10× the resistance of the lower resistance portion.

In some examples, the conductive portion comprises a current pathway comprising two lower resistance portions with a higher resistance portion in-between, wherein the lower resistance portions each have a pathway width that is at least 2, 3, 4, 5, 6, 7, 8, 9 or 10× the pathway width of the higher resistance portion.

In some examples, the conductive portion comprises on the first surface:
a. an inner, higher resistance portion, for generating aerosol from aerosol-generating material by resistance heating; and
b. a pair of outer, lower resistance portions, one either side of the inner, higher resistance portion, for supplying power thereto.

In some examples, the lower resistance portion(s) extend from the first surface of the aerosol generator to the second surface of the aerosol generator.

In some examples, the outer and/or lower resistance portions extend from a first surface of the aerosol generator to multiple other surfaces of the aerosol generator, which may be perpendicular.

In some examples, the outer and/or lower resistance portions extend from the first surface of the aerosol generator to the second surface of the aerosol generator, substantially forming an L-shape.

In some examples, only the outer and/or lower resistance portions extend from the first surface of the aerosol generator to the second surface of the aerosol generator. In other words, in some examples, the inner portion may not extend from the first surface of the aerosol generator to the second surface of the aerosol generator.

In some examples, the outer and/or lower resistance portions are substantially rectangular. In some examples, the outer and/or lower resistance portions have a length extending from the first surface of the aerosol generator to the second surface of the aerosol generator.

In some examples, the system or cartridge further comprises an electrically conductive element for connecting the conductive portion to a power supply.

In some examples, the conductive portion is exposed to an air flow path from an air inlet to the aerosol generator and the electrically conductive element does not protrude into the air flow path.

In some examples, further comprising an electrically conductive element for connecting the conductive portion to a power supply.

In some examples, the electrically conductive element contacts the conductive portion on the second surface.

In some examples, the conductive portion is exposed to an air flow path from an air inlet to an aerosol outlet and the electrically conductive element does not protrude into the air flow path.

In some examples, the electrically conductive element has an axis of extent along a length that is substantially parallel to an axis of extent along a length of an airflow path from an air inlet to an aerosol outlet.

In some examples, the system or cartridge comprises an air flow path past the conductive portion for entraining aerosol generated by the aerosol generator into air from an air inlet. In some examples, the conductive portion is exposed to the air flow path and the conductive element does not protrude beyond the conductive portion towards the air flow path.

In some examples, the conductive element is substantially flush with the conductive portion exposed to the air flow path.

In some examples, the conductive element is not exposed or not directly exposed to the air flow path.

In some examples, the conductive element does not extend into the air flow path. In some examples, the conductive element does not extend directly into a linear air flow path.

In some examples, the system or cartridge comprises a barrier between the conductive element and the air flow path. In some examples, the barrier comprises a tube.

In some examples, the aerosol generator comprises a body between the conductive element and the conductive portion, spacing the conductive element from the conductive portion.

In some examples, the electrically conductive element does not protrude towards the air flow path beyond a higher resistance portion of the conductive portion.

In some examples, the conductive portion extends from a first, vaporisation surface of the aerosol generator configured to generate vapour, to a second, parallel surface configured to connect the conductive portion to a power supply.

In some examples, the conductive portion extends internally and/or externally between surfaces.

In some examples, the first surface comprises a vaporisation surface that is:
a. adjacent to an airflow path from an air inlet to an aerosol outlet; and/or
b. substantially parallel to an air flow path past the vaporisation surface.

In some examples, the first surface comprises a vaporisation surface having a width or length that is substantially parallel to an air flow path past the vaporisation surface. In other words, in some examples, air flow past the first, vaporisation surface conductive portion is substantially parallel to the 2D planar extent of the first, vaporisation surface conductive portion.

In some examples, comprising an aerosol outlet at least partially axially aligned with an air inlet, providing a substantially straight flow path from the inlet to the outlet.

In some examples, the aerosol outlet is fully axially aligned with the air inlet.

In some examples:
a. the aerosol generator comprises a porous body, configured to transport aerosol-generating material to the conductive portion for aerosolisation, wherein the first and second surfaces are planar and substantially perpendicular to one another;
b. the conductive portion comprises:
   i. a higher resistance portion configured to generate aerosol from aerosol-generating material by resistance heating; and
   ii. a lower resistance portion, configured to supply power to the higher resistance portion; and
c. the system comprises an air inlet and an aerosol outlet, wherein the aerosol outlet is at least partially axially aligned with the air inlet, providing a substantially straight flow path from the air inlet, past the higher resistance portion, to the aerosol outlet, wherein the flow path is substantially parallel to the width or length of the higher resistance conductive portion.

In some examples, the conductive element is a unitary conductive element. In some examples, the conductive element comprises brass, optionally comprising nickel, silver and/or gold plating.

The conductive element(s) may be in contact with the conductive portion but not fixed thereto, or be in contact with and fixed to the conductive portion, e.g. by soldering. In some examples, the conductive element(s) contact the first or second surface conductive portions of the aerosol generator exclusively, or contacts multiple surface conductive portions.

In some examples, the electrically conductive element comprises one or a pair of pogo pins.

In some examples, the aerosol generator comprises a wicking element and an external surface of the wicking element comprises the conductive portion. In some examples, the wicking element comprises a ceramic material. In some examples, the wicking element comprises cotton.

In some examples, the conductive portion is at least partially embedded in or fused in the external surface. In some examples, the conductive portion has a maximum thickness of ≤ 0.5 mm, ≤ 0.25 mm, ≤ 0.1 mm or ≤ 0.05 mm.

In some examples, the aerosol delivery system comprises a pair of conductive elements spaced either side of an air inlet or airflow pathway to the aerosol generator. In some examples, the aerosol delivery system comprises a pair of the conductive elements configured to connect terminals on opposing sides or outer portions of the conductive portion to respective positive and negative terminals of a power supply.

In some examples the conductive portion is configured to provide a heating zone between the positive and negative terminals in use.

In some examples, the conductive portion is adjacent to an airflow path from an air inlet to an aerosol outlet. In some examples, the conductive portion is directly exposed to airflow in the airflow pathway. In some examples, the conductive portion extends into the airflow pathway. In some examples, the conductive portion is downstream of the air inlet.

In some examples, the aerosol delivery system is or comprises a cartridge for aerosol-generating material.

In some examples, the aerosol delivery system further comprises:
a mouthpiece; and/or
a controller for controlling supply of power to the aerosol generator; and/or
a power supply.

### Brief description of the figures

Embodiments of the disclosure will now be described, by way of example only, with reference to the accompanying drawings, in which:
Figure 1 is a schematic cross-section view of an aerosol delivery system comprising an aerosol generator;
Figure 2 is a schematic side view of an aerosol delivery system comprising an aerosol generator;
Figure 3 is a schematic inverted perspective view of the system of figure 2;
Figure 4 is a schematic side view of another aerosol delivery system comprising an aerosol generator;
Figure 5 is a schematic perspective view of the system of figure 4;
Figures 6a-6c are schematic perspective views of various aerosol generators comprising a multi-surface conductive portion;
Figure 6d is a schematic cross-section view of another aerosol generator comprising a multi-surface conductive portion;
Figures 7a-7b are schematic perspective views of the aerosol generator of figure 6a;
Figure 7c is a schematic perspective view of the aerosol generator of figure 6b;
Figures 8a and 8b are schematic perspective views of systems comprising the aerosol generator of figure 6a;
Figures 9a-9c are schematic cross-section views of further systems comprising an aerosol generator having a multi-surface conductive portion;
Figures 10a-10b are schematic cross-section views of systems comprising an aerosol generator having a curved conductive portion; and
Figure 11 is a schematic cross-section view of another system comprising an aerosol generator.

### Detailed description of the disclosure

Aspects and features of certain examples and embodiments are described herein. Some aspects and features may be implemented conventionally and these are not described in detail, for brevity.

The claimed invention may generally provide a sub-assembly or sub-system suitable for use in an aerosol delivery system, or configured for use in an aerosol delivery system. The sub-system may generally form part of an aerosol delivery system and in particular may form part of the reusable device and/or the consumable cartridge of a two-part system.

### Introduction

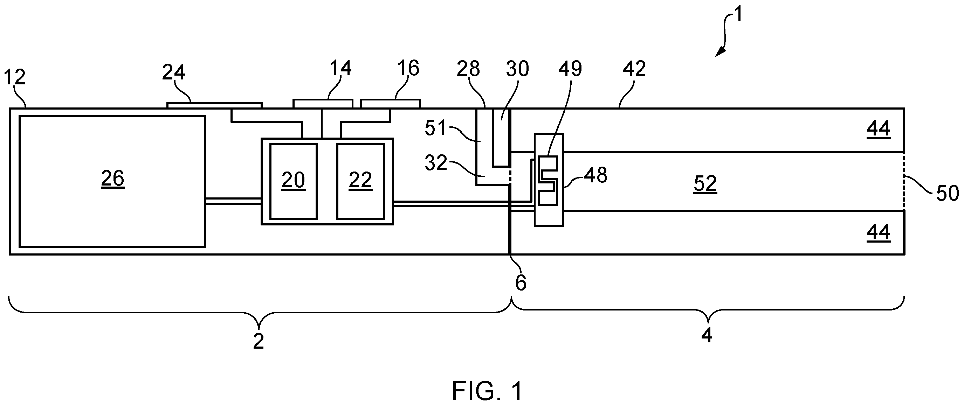

Figure 1 is a cross-sectional view through an example aerosol delivery system 1 in accordance with certain embodiments of the disclosure, providing an introduction to two-part aerosol delivery systems, the components therein and their functionality.

The aerosol delivery system 1 comprises two main parts, a reusable part 2 (sometimes referred to as a control unit) and a replaceable / disposable consumable cartridge part 4 (sometimes referred to as a consumable or an article). In normal use, the reusable part 2 and the cartridge part 4 are releasably coupled together at an interface 6. When the cartridge part 4 is exhausted or the user wishes to switch to a different cartridge part 4, the cartridge part 4 may be removed from the reusable part 2 and a replacement cartridge part 4 attached to the reusable part 2 in its place. The interface 6 may provide a structural, electrical and airflow path connection between the two parts 2, 4 and may be established in accordance with conventional techniques, e.g. based around a screw thread, magnetic or bayonet fixing with electrical contacts and openings for the electrical connection and airflow path between the two parts 2, 4 as appropriate. The specific manner by which the cartridge part 4 mounts to the reusable part 2 is not significant to the principles described herein, but is assumed here to comprise a magnetic coupling (not represented in figure 1). It will also be appreciated the interface 6 in some implementations may not support an electrical and / or airflow path connection between the respective parts 2, 4. For example, in some implementations an aerosol generator may be provided in the reusable part 2 rather than in the cartridge part 4, or the transfer of electrical power from the reusable part 2 to the cartridge part 4 may be wireless (e.g. based on electromagnetic induction), so that an electrical connection between the reusable part 2 and the cartridge part 4 is not needed. Furthermore, in some implementations the airflow through the system 1 might not go through the reusable part 2, so that an airflow path connection between the reusable part 2 and the cartridge part 4 is not needed. In some instances, a portion of the airflow path may be defined at the interface between portions of the reusable part 2 and cartridge part 4 when these are coupled together for use.

The cartridge / consumable part 4 may, in certain embodiments, be broadly conventional. In figure 1, the cartridge part 4 comprises a cartridge housing 42 formed of a plastics material. The cartridge housing 42 supports other components of the cartridge part 4 and provides the mechanical interface 6 with the reusable part 2. The cartridge housing 42 is generally circularly symmetrical about a longitudinal axis along which the cartridge part 4 couples to the reusable part 2. In this example, the cartridge part 4 has a length of around 4 cm and a diameter of around 1.5 cm. However, the specific dimensions, geometry, overall shapes and materials used may vary.

Within the cartridge housing 42 is a chamber or reservoir 44 that contains aerosol-generating material. In the example of figure 1, the reservoir 44 stores a supply of liquid aerosol generating material and the liquid reservoir 44 has an annular shape with an outer wall defined by the cartridge housing 42 and an inner wall that defines an airflow path 52 through the cartridge part 4. The reservoir 44 is closed at each end with end walls to contain the aerosol generating material. The reservoir 44 may be formed conventionally, e.g. comprising a plastics material and/or integrally moulded with the cartridge housing 42.

The cartridge / consumable part 4 further comprises an aerosol generator 48, which in this example is located towards an end of the reservoir 44, opposite to a mouthpiece outlet 50. In a two-part system such as in figure 1, the aerosol generator 48 may be in either of the reusable part 2 or the cartridge part 4. For example, in some embodiments, the aerosol generator 48 (e.g. a heater, which may be in the form of a wick and coil arrangement, a distiller, e.g. formed from a sintered metal fibre material or other porous conducting material, a ceramic porous body comprising a conductive portion 49 as shown, or any suitable alternative aerosol generator) may be comprised in the reusable part 2, and is brought into proximity with a portion of aerosol generating material in the cartridge part 4 when the cartridge part 4 is engaged with the reusable part 2.

In embodiments, the aerosol generator 48 has an electrically conductive portion extending from a first surface of the aerosol generator 48 to a second, different surface of the aerosol generator 48, as is described further later, with reference to the subsequent figures.

In the example of figure 1, a wick 46 in contact with the aerosol generator 48 extends transversely across the cartridge airflow path 52 with its ends extending into the reservoir 44 of the liquid aerosol generating material through openings in the inner wall. The openings in the inner wall of the reservoir 44 are sized to broadly match the dimensions of the wick 46 to provide a reasonable seal against leakage from the reservoir 44 into the cartridge airflow path 52, without unduly compressing the wick 46, which may be detrimental to its fluid transfer performance.

The wick 46 and aerosol generator 48 are arranged in the cartridge airflow path 52 such that a region of the cartridge airflow path 52 around the wick 46 and heater 48 in effect defines a vaporisation region for the cartridge part 4. Aerosol generating material in the reservoir 44 infiltrates the wick 46 through the ends of the wick extending into the reservoir 44 and is drawn along the wick by surface tension / capillary action (i.e. wicking). In use, electrical power may be supplied to the aerosol generator 48 to vaporise an amount of aerosol generating material drawn to the vicinity of the aerosol generator 48 by the wick 46. Vaporised aerosol generating material may then become entrained in air drawn along the cartridge airflow path from the vaporisation region towards the mouthpiece outlet 50 for user inhalation.

As noted above, the rate at which aerosol generating material is vaporised by the aerosol generator 48 will depend on the amount (level) of power supplied to the aerosol generator 48. Thus, electrical power can be applied to the aerosol generator 48 to selectively generate aerosol from the aerosol generating material in the cartridge part 4, and furthermore, the rate of aerosol generation can be changed by changing the amount of power supplied to the aerosol generator 48, for example through pulse width and/or frequency modulation techniques.

The reusable part 2 comprises an outer housing 12 having an opening that defines an air inlet 28 for the system 1, a power source 26 (e.g. a battery) for providing operating power for the system 1, control circuitry / controller 22 for controlling and monitoring the operation of the system 1, a first user input button 14, a second user input button 16, and a visual display 24. The outer housing 12 may be formed, e.g. from a plastics or metallic material and in this example has a circular cross section generally conforming to the shape and size of the cartridge part 4, to provide a smooth transition between the two parts 2, 4 at the interface 6. In this example, the reusable part 2 has a length of around 8 cm so the overall length of the system 1 when the cartridge part 4 and the reusable part 2 are coupled together is around 12 cm. However, the specific dimensions, geometry, overall shapes and materials used may vary.

The air inlet 28 connects to an airflow path 51 through the reusable part 2. The reusable part airflow path 51 in turn connects to the cartridge airflow path 52 across the interface 6 when the reusable part 2 and cartridge part 4 are connected together. Thus, when a user inhales on the mouthpiece opening 50, air is drawn in through the air inlet 28, along the reusable part airflow path 51, across the interface 6, through the aerosol generation area in the vicinity of the aerosol generator 48 (where vaporised aerosol generating material becomes entrained in the air flow), along the cartridge airflow path 52, and out through the mouthpiece opening 50 for user inhalation.

The power source 26 in this example is rechargeable and may be a conventional type, e.g. of the kind normally used in electronic cigarettes and other applications requiring provision of relatively high currents over relatively short periods. The power source 26 may be recharged through a charging connector in the reusable part housing 12, for example a USB connector.

Optionally, first and/or second user input buttons 14, 16 may be provided, which in this example are conventional mechanical buttons, e.g. comprising a spring mounted component which may be pressed by a user to establish an electrical contact. The input buttons may be input devices for detecting user input and the manner in which the buttons are implemented is not significant. The buttons may be assigned functions such as switching the system 1 on and off, and/or adjusting user settings such as a power to be supplied from the power source 26 to the aerosol generator 48.

A display 24 may be provided to give a user a visual indication of various characteristics associated with the aerosol delivery system, e.g. current power setting information, remaining power source power, etc. The display may be implemented in various ways. In this example, the display 24 comprises a conventional pixilated LCD screen. In other implementations, the display may comprise one or more discrete indicators, e.g. LEDs, arranged to display information, e.g. through particular colours and/or flash sequences. More generally, the manner in which the display 24 is provided and information is displayed is not significant to the principles described herein - other embodiments may not include a visual display and/or may include other means for providing a user with information relating to operating characteristics of the system 1, e.g. using audio signalling.

A controller 22 is suitably configured / programmed to control the aerosol delivery system 1 to provide functionality as described herein, as well as for providing conventional operating functions of the system 1. The controller (processor circuitry) 22 may be considered to logically comprise various subunits / circuitry elements associated with different aspects of the operation of the system 1. In this example, the controller 22 comprises power supply control circuitry for controlling the supply of power from the power source 26 to the aerosol generator 48 in response to user input, user programming circuitry 20 for establishing configuration settings (e.g. user-defined power settings) in response to user input, as well as other functional units / circuitry associated functionality in accordance with the principles described herein and conventional operating aspects, such as display driving circuitry and user input detection circuitry. The functionality of the controller 22 can be provided in various different ways, e.g. using one or more programmed programmable computer(s) and / or one or more suitably configured application-specific integrated circuit(s) / circuitry / chip(s) / chipset(s). The controller 22 may comprise an application specific integrated circuit (ASIC), CPU, microprocessor or microcontroller. The operations of a controller and other electronic components are generally controlled by software/instructions running on the controller, which may be stored in non-volatile memory, (e.g. ROM), which may be integrated into the controller, or provided separately. The controller 22 may access the ROM to load and execute individual software as and when required.

The reusable part 2 comprises an airflow sensor 30, which is electrically connected to the controller 22. In most embodiments, the airflow sensor 30 comprises a so-called "puff sensor", in that the airflow sensor 30 is used to detect when a user is puffing on the system 1. In some embodiments, the airflow sensor 30 comprises a switch in an electrical path providing electrical power from the power source 26 to the aerosol generator 48. In such embodiments, the airflow sensor 30 generally comprises a pressure sensor configured to close the switch when subjected to a particular range of pressures, enabling current to flow from the power source 26 to the aerosol generator 48 once the pressure in the vicinity of the airflow sensor 30 drops below a threshold value. The threshold value can be set to a value determined by experimentation to correspond to a characteristic value associated with the initiation of a user puff. In other embodiments, the airflow sensor 30 is connected to the controller 22, and the controller 22 distributes electrical power from the power source 26 to the aerosol generator 48 in dependence of a signal received from the airflow sensor 30 by the controller 22. The specific manner in which the signal output from the airflow sensor 30 (which may comprise a measure of capacitance, resistance or other characteristic of the airflow sensor, made by the controller 22) is used by the controller 22 to control the supply of power from the power source 26 to the aerosol generator 48 can be carried out in accordance with any approach known to the skilled person.

In the example shown in figure 1, the airflow sensor 30 is mounted to an optional printed circuit board (PCB). The airflow sensor 30 may comprise any sensor configured to determine a characteristic of airflow in an airflow path 51 disposed between air inlet 28 and mouthpiece opening 50, e.g. a pressure sensor or transducer (such as a membrane or solid-state pressure sensor), a combined temperature and pressure sensor, or a microphone (e.g. an electret-type microphone), which is sensitive to changes in air pressure, including acoustical signals. The airflow sensor 30 is situated within a sensor cavity or chamber 32, which comprises the interior space defined by one or more chamber walls. The sensor cavity 32 comprises a region internal to one or more chamber walls in which an airflow sensor 30 can be fully or partially situated. In some embodiments, the PCB comprises one of the chamber walls of a sensor housing comprising the sensor chamber / cavity 32.

A deformable membrane may be disposed across an opening communicating between the sensor cavity 32 containing the sensor 30, and a portion of the airflow path disposed between air inlet 28 and mouthpiece opening 50. The deformable membrane covers the opening, and is attached to one or more of the chamber walls according to approaches described further herein.

The aerosol delivery system 1 may comprise communication circuitry configured to connect to one or more further electronic devices (e.g., a storage / charging case, or a refill / charging dock) to enable data transfer between the system 1 and further electronic device(s). The communication circuitry may be integrated into the controller 22, or implemented separately. The communication circuitry may be configured to support wired or wireless communications between the aerosol delivery system 1 and other electronic devices such as a case, a dock, a computing device such as a smartphone or PC, a base station supporting cellular communications, a relay node providing an onward connection to a base station, a wearable device, or any other portable or fixed device. The controller 22, other components within the system 1 and other devices/systems may comprise one or more processors and data processing may be performed on any of these processors or on a remote processor, the data communicated by wire or wirelessly.

Wireless communications between the aerosol delivery system 1 and a further electronic device may be configured according to data transfer protocols such as Bluetooth^{®}, ZigBee, WiFi^{®}, Wifi Direct, GSM, 2G, 3G, 4G, 5G, LTE, NFC, RFID, or generally any other wireless, and/or wired, network protocol or interface. The communication circuitry may comprise any suitable interface for wired data connection, such as USB-C, micro-USB or Thunderbolt interfaces, and may comprise pin or contact pad arrangements configured to engage cooperating pins or contact pads on a dock, case, cable, or other external device which can be connected to the aerosol delivery system 1.

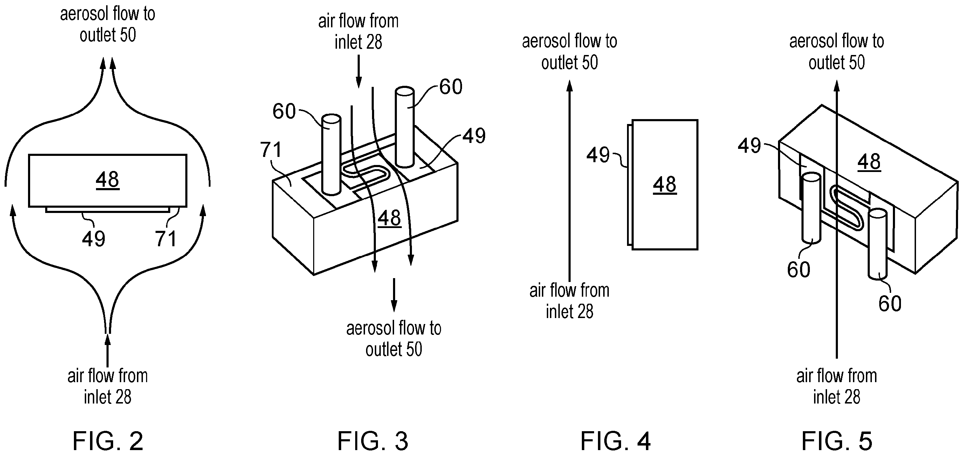

Figures 2-5 illustrate particular configurations of an aerosol generator 48 in a flow path from an air inlet 28 to an aerosol outlet 50.

Figure 2 is a side view of an aerosol delivery system comprising an aerosol generator 48 having a porous (e.g. ceramic) body functioning as a wick, configured to receive aerosol-generating material from a reservoir 44 in use (not shown). The aerosol generator 48 comprises a substantially planar electrically conductive portion 49 on a first surface 71 of the aerosol generator 48, for generating aerosol from aerosol-generating material, e.g. using resistance heating. In figure 2, the conductive portion 49 faces head-on into and obstructs a flow path from an air inlet 28 to an aerosol outlet 50 (also shown in figure 3). Air flow through the air inlet 28 flows towards the conductive portion 49 obstructing the flow path and so is diverted around the conductive portion 49 and the aerosol generator 48 to the outlet 50.

Figure 3 is an inverted perspective view of the system of figure 2, additionally illustrating a pair of conductive elements 60 contacting the conductive portion 49, for connecting the conductive portion 49 to a power source 26, to supply power to the conductive portion 49 and aerosolise aerosol-generating material in use. In figure 3, the conductive elements 60 have the same axis of extent towards the conductive portion 49 as the air flow path from the inlet 28 to the outlet 50 and the conductive elements 60 are exposed to the air flow path.

Figure 4 is a side view of another aerosol delivery system comprising an aerosol generator 48 having a porous body and configured to receive aerosol-generating material from a reservoir 44 in use (not shown). By contrast to figure 2, the substantially planar electrically conductive portion 49 remains exposed to, but here is aligned with, the flow path from the air inlet 28 to the outlet 50. This example provides a straight flow path from the inlet 28 to the outlet 50, past the conductive portion 49.

Figure 5 is a perspective view of the system of figure 4, again additionally illustrating a pair of conductive elements 60 contacting the conductive portion 49 to supply power to the conductive portion 49 and aerosolise aerosol-generating material in use. In figure 5, the conductive elements 60 again have the same axis of extent towards the conductive portion 49 as the flow path from the inlet 28 to the outlet 50, but by contrast to figure 3, the flow path is now parallel past the conductive portion 49. The arrangement of figures 4-5 provides a more direct, straight flow path for vapour generated by the aerosol generator 48 and transported to the user via the outlet 50. The conductive elements 60 remain exposed to the air flow path and make contact at a vertical interface between the conductive elements 60 and the conductive portion 49

### Multi-surface aerosol generator conductive portion

As outlined above, some embodiments of the claimed invention comprise an aerosol generator 48 having an electrically conductive portion 100 for generating aerosol from aerosol-generating material in use, wherein the conductive portion 100 extends from a first surface 71 of the aerosol generator 48 to a second, different surface 72 of the aerosol generator 48. Accordingly, such embodiments comprise a multi-surface electrically conductive portion 100.

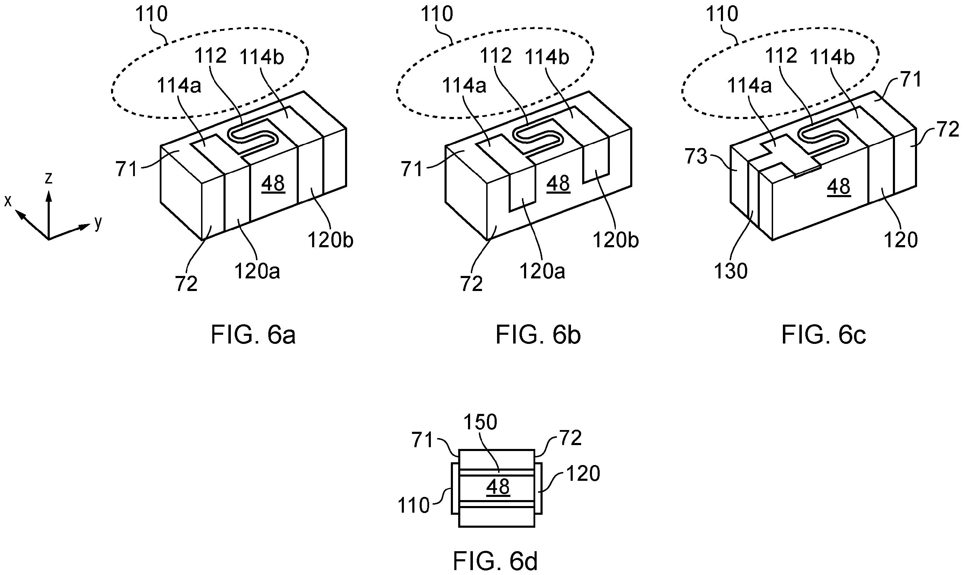

Figures 6a-6c are perspective views of various aerosol generators 48 comprising a multi-surface aerosol generator conductive portion 100. In figures 6a-6c, the aerosol generator 48 is a rectangular porous body, e.g. comprising ceramic or cotton, for retaining and/or transporting aerosol-generating material, e.g. from a reservoir 44, optionally via a wick 46, to the conductive portion 100. In some examples, the conductive portion 100 generates aerosol by resistance heating of the aerosol-generating material. Conductive portions detailed herein may generally take any form, e.g. comprise a trace, thin-film or etched conductive portion, or a mesh. In some examples, the conductive portion is at least partially embedded in or fused in an external surface of the aerosol generator 48. The conductive portion may be very thin, such as a coating, particularly notably thinner than the aerosol generator 48, and thus references to relative angles between surfaces of the aerosol generator 48 may generally be assumed to apply equally to angles between conductive portions on those surfaces, unless otherwise stated. In some examples, the aerosol generator 48 is approximately 2-5 mm wide x 2-5 mm deep, preferably square in cross-section, and 5-15 mm long. In some examples, the conductive portion has a maximum thickness of ≤ 0.5 mm, ≤ 0.25 mm, ≤ 0.1 mm or ≤ 0.05 mm.

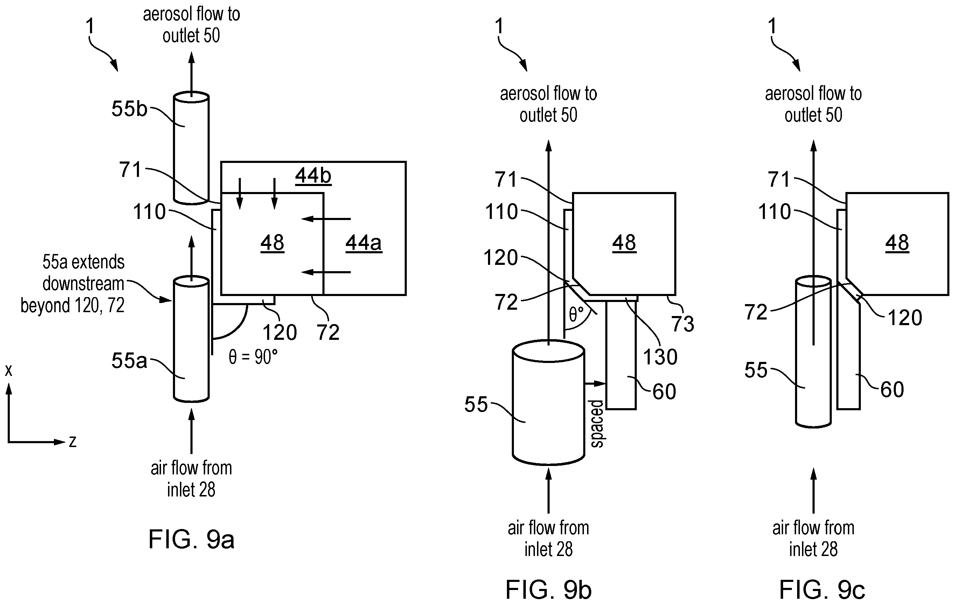

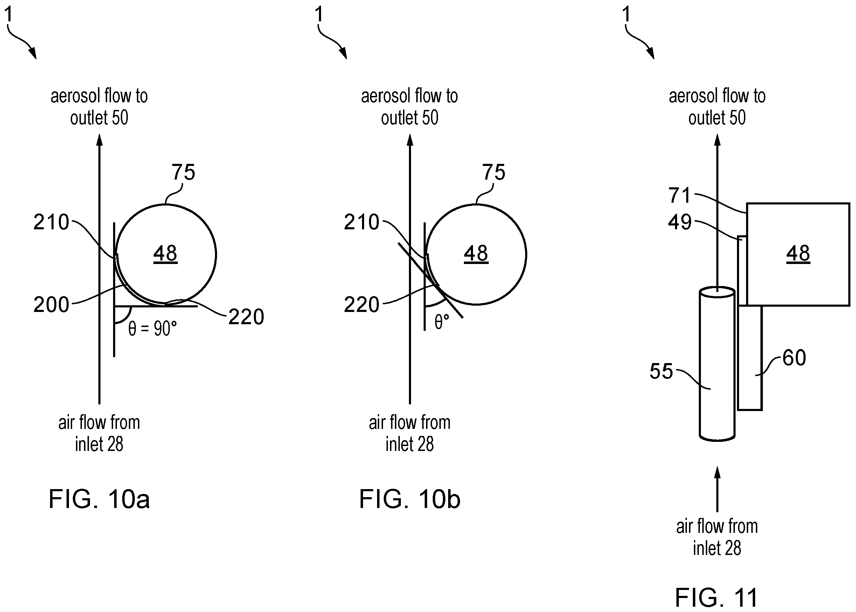

In figures 6a-6c, the conductive portion 100 comprises a first surface conductive portion 110 on the first surface 71 of the aerosol generator 48 and a second surface conductive portion 120 on the second surface 72 of the aerosol generator 48. In figure 6c, the conductive portion 100 additionally comprises a third surface conductive portion 130 on a third surface 73 of the aerosol generator 48. In these examples, the first 71, second 72 and third 73 surfaces (where present) are substantially planar, adjacent to and perpendicular to one another, but this may not be the case in other examples. In particular, other examples with non-perpendicular first and second surfaces 71, 72; or a curved surface 75, are described later, with reference to figures 9b-9c and 10a-10b.

The first surface 71 may comprise a vaporisation surface, configured to receive aerosol-generating material and generate vapour/aerosol therefrom, e.g. by resistance heating of the first, vaporisation surface conductive portion 110. The second and/or third surfaces 72, 73 may comprise a power supply surface configured to receive power from a power supply 26 at power supply surface conductive portion(s) 120, 130 and conduct power to the first, vaporisation surface conductive portion 110. The power supply surface(s) 72, 73 as a whole, the conductive portion(s) 120, 130 thereof or parts of the surfaces 72, 73 corresponding to the conductive portion(s) 120, 130 may be configured not to receive aerosol-generating material, e.g. the system 1 or more specifically the aerosol generator 48 may comprise a barrier, such as an impermeable coating or layer, to minimise or prevent transfer/receipt of aerosol-generating material to the power supply conductive portion(s) 120, 130, or the surfaces 72, 73 or conductive portion(s) 120, 130 thereof may have a suitably small pore size to minimise transfer/leakage of the aerosol-generating material. Generally, the conductive portions 110, 120, 130, 200 on any given surface 71, 72, 73, 75 may comprise multiple parts, e.g. as described further below.

In figure 6a, the first surface conductive portion 110 is substantially planar (although may have a small variation in thickness) and comprises a first surface inner conductive portion 112, here substantially 'S' - shaped, and two rectangular first surface outer conductive portions 114a, 114b, one either side of and connected to the first surface inner conductive portion 112. In figure 6a, the outer conductive portions 114a, 114b are spaced / inset from an edge of the first surface 71 that is distal from the second surface 72, and extend across to the second surface 72.

In some examples, as shown in figures 6-8, conductive portions described herein may generally comprise a higher resistance portion or part and a lower resistance portion or part. Beneficially, this may permit concentrating aerosol generation in particular regions of the aerosol generator 48 (e.g. when generating aerosol by resistance heating, higher resistance regions will generate more heat, forming a concentrated heating zone, thus a surface comprising a higher resistance conductive portion may be considered a vaporisation surface/portion, since this will generate more vapour than a lower resistance portion). In particular, the conductive portion may comprise a higher resistance portion, for generating aerosol from aerosol-generating material by resistance heating and a lower resistance portion, for supplying power to the higher resistance portion. The higher resistance portion may be located between a pair of lower resistance portions. In some examples, the higher resistance portion has a resistance that is at least 2, 3, 4, 5, 6, 7, 8, 9 or 10× the resistance of the lower resistance portion. The dimensions of the conductive portion may be adjusted to maintain resistance within a reasonable range and e.g. provide the same overall resistance for single surface, multi-surface and curved surface conductive portions 49, 100, 200.

In the examples of figures 6-8, the first surface conductive portion 110 comprises a current pathway comprising two lower resistance outer conductive portions 114 with a higher resistance inner conductive portion 112 in-between. Any lower resistance conductive portions may extend from the first surface 71 onto one or more other (e.g. second 72, third 73) surfaces of the aerosol generator 48, thus any second surface conductive portions 120 may comprise lower resistance portions relative to a higher resistance portion. One or more of the lower resistance portions may have a pathway width that is at least 2, 3, 4, 5, 6, 7, 8, 9 or 10× the pathway width of the higher resistance portion.

In the examples of figures 6a-6b, the first surface outer conductive portions 114 are rectangular, having the same pathway width, and a length extending from the first surface 71 to the second surface 72. The overall conductive portion 100, here the lower resistance portions thereof, (excluding the higher resistance inner portion 112) may comprise a pair of 'L'-shapes across the first and second surfaces 71, 72, as shown.

In figure 6a, the second surface conductive portion 120 is also substantially planar (but again might have a small variation in thickness) and comprises two portions 120a, 120b, both having the same profile as, and forming a linear extension across the second surface 72 of, the first surface outer conductive portions 114a, 114b. The two second surface conductive portions 120a, 120b each extend across a full span of the second surface 72. Although not shown, electrically conductive elements 60a, 60b may be provided to supply power from a power source 26 to the conductive portion 100 and specifically to the first surface conductive portion 110 and/or any higher resistance part thereof, via the respective second surface conductive portions 120a, 120b.

The arrangement of figure 6b is similar to that of figure 6a, save for the two second surface conductive portions 120a, 120b not extending across a full span of the second surface 72 - in figure 6b, they extend approximately 60% across the span (width). Beneficially, this may reduce the material cost and time for forming the conductive portion 100.

In figure 6c, the conductive portion 100 extends from the first surface 71 to second 72 and third 73 different surfaces of the aerosol generator 48. In this example, the first 71, second 72 and third 73 surfaces are all adjacent and mutually perpendicular. By contrast to the figure 6a example, a first surface outer conductive portion 114a is 'T'-shaped and extends to both the second and a third surface 73 of the aerosol generator 48. The third surface conductive portion 130 comprises a rectangular portion spanning a full edge-to-edge width of the third surface 73. The other first surface outer conductive portion 114b and second surface conductive portion 120 are as shown in figure 6a. In another example, not shown, the conductive portion 100 extends from the first surface 71 to second 72 and third 73 different surfaces of the aerosol generator 48, where the third surface 73 opposes the second surface 72.

Figure 6d is a cross-section view of another aerosol generator 48 comprising a multi-surface aerosol generator conductive portion 100. In contrast to figures 6a-6c, the first and second surfaces 71, 72 are parallel and oppose one another, spaced from one other by the aerosol generator body and another (third) surface. The conductive portion 100 comprises first and second surface conductive portions 110, 120 and an interconnecting internal conductive portion 150 extending internally through the aerosol generator body between the first 71 and second 72 surfaces of the aerosol generator 48, to provide an electrically conductive pathway between the first and second surface conductive portions 110, 120. Beneficially, in this arrangement, if the aerosol generator 48 retains aerosol-generating material proximal to the internal conductive portion 150 then it may be pre-heated by the internal conductive portion 150. Furthermore, a conductive element 60 (not shown) contacting the second (power supply) surface conductive portion 120 is then spaced from the first surface conductive portion 110, so the conductive element 60 is not exposed to air flow past the first surface conductive portion 110.

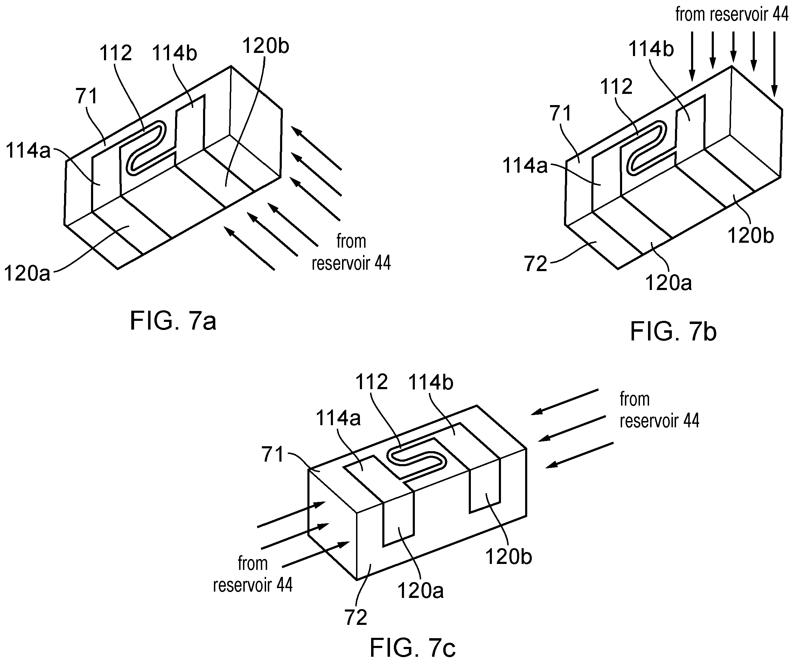

Figures 7a-7b are perspective views of the aerosol generator 48 of figure 6a showing the transport of aerosol-generating material from a reservoir 44 (not shown) to the conductive portion 100, for aerosolisation. In figure 7a, the reservoir 44 is on a back surface of the aerosol generator 48, parallel to and opposing the first, vaporization surface 71. As shown by the arrows, aerosol-generating material is delivered from the reservoir 44 to a (back) surface of the aerosol generator that opposes the first, vaporization surface 71, and is transported to the first, vaporisation surface 71 by capillary action through the porous aerosol generator body. In figure 7b, the reservoir 44 is on a top surface of the aerosol generator 48, where the top surface is both perpendicular to the first, vaporization surface 71 and parallel to and opposing the power supply surface 72 of the aerosol generator 48. Aerosol-generating material is transported from the reservoir 44 to the first, vaporisation surface 71 by capillary action, aided by gravity in this orientation (where the first, vaporisation surface 71 is orientated as a vertical plane, or, in other words, have a depth extending horizontally). As such, in this orientation, the reservoir 44 of aerosol-generating material is vertically above the aerosol generator 48 and configured to deliver aerosol-generating material under the influence of gravity to the top surface of the aerosol generator 48.

Figure 7c is a perspective view of the aerosol generator 48 of figure 6b, showing the transport of aerosol-generating material from a side reservoir 44 (not shown) to the conductive portion 100, for aerosolisation. In figure 7c, the reservoir 44 contacts two opposing side surfaces of the aerosol generator 48 akin to the arrangement in figure 1, the side surfaces being perpendicular to both the first, vaporization surface 71 and the second, power supply surface 72.

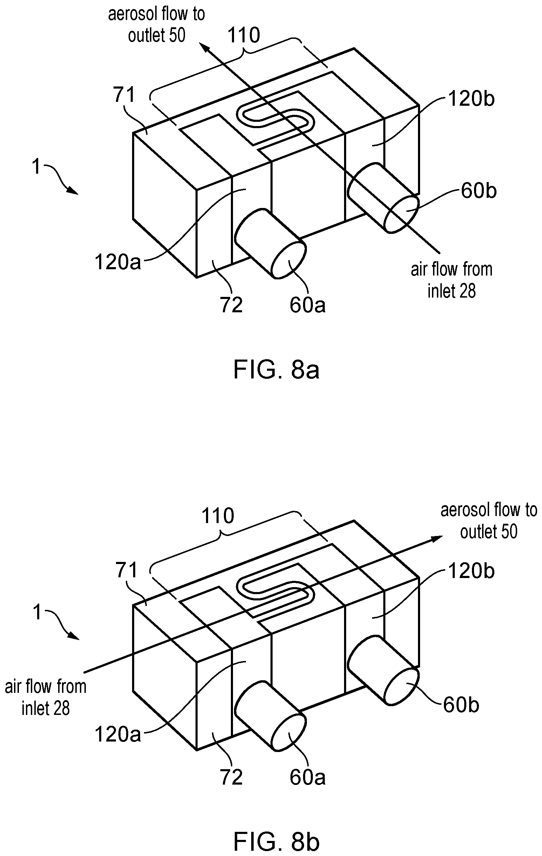

Figures 8a and 8b are perspective views of systems 1 comprising the aerosol generator 48 of figure 6a with first and second electrically conductive elements 60a, 60b in electrical contact with the respective second surface conductive portions 120a, 120b, for supplying power to the first surface conductive portion 110. Figures 8a and 8b illustrate a flow path past the first surface conductive portion 110, for entraining aerosol generated by the aerosol generator 48 into air from an air inlet 28 for delivery to an aerosol outlet 50, wherein the conductive portion 100 is directly exposed to the air flow path. More specifically, the first, vaporization surface 71, comprising the first surface conductive portion 110, extends in a plane that is parallel to the air flow path, whilst the second, power supply surface 72, comprising the second surface conductive portion 120 extends in a plane that is perpendicular to the air flow path. In these examples, both the first and second surfaces 71, 72 and their respective conductive portions 110, 120 are exposed to the flow path.

The conductive element(s) 60 themselves may be unitary, comprise a pogo pin and/or may comprise brass, optionally comprising nickel, silver and/or gold plating. The conductive element(s) 60 may be in contact with the conductive portion 100 but not fixed thereto, or be in contact with and fixed to the conductive portion 100, e.g. by soldering. In some examples, the conductive element(s) 60 contact the first or second (power supply) surface conductive portion 110, 120 of the aerosol generator 48 exclusively, or contact multiple surface conductive portions 110, 120, 130. Examples may comprise a pair of conductive elements 60 configured to connect terminals on opposing ends or outer portions of the conductive portion 100 (particularly of a second, power supply surface conductive portion 120) to respective positive and negative terminals of a power supply 26.

In these examples, the conductive elements 60 beneficially do not protrude beyond the conductive portion 100 towards the air flow path (particularly not beyond the first surface conductive portion 110 which extends in a plane that is parallel to the air flow path), and in some examples may not be exposed to the air flow path at all. In figure 8a, the electrically conductive elements 60 have an axis of extent along a length that is substantially parallel to an axis of extent along a length of the flow path. In figure 8b, by contrast, the electrically conductive elements 60 have an axis of extent along a length that is substantially perpendicular to an axis of extent along a length of the flow path. In both figures 8a and 8b, the conductive elements 60 are spaced away from the vaporization surface 71 comprising the first (vaporisation) surface conductive portion 110, which is directly exposed to the flow path. A barrier, such as a tube 55 for fluid flow, may be provided to minimise or avoid exposing one or more of the second, power supply surface 72 and/or the second surface conductive portion(s) 120 and/or the conductive element(s) 60 to the flow path. Avoiding exposure of the conductive elements 60 to the flow path in particular is beneficial to aid delivery of larger vapour droplets to the user, since the air flow is not disturbed by the conductive elements 60, and this may avoid undesirable contamination of the vapour/aerosol generation with material from the conductive elements 60.

Although the conductive portions in figures 8a and 8b comprise multi-surface aerosol generator conductive portions 100, this is not essential and instead the conductive portion may comprise a curved conductive portion 200 and/or extend across only a single surface of the aerosol generator 48, e.g. as per the conductive portion 49 in figures 2-5. Such examples are described below with reference to figure 11.

Figures 9a-9c are cross-section views of further systems 1 comprising an aerosol generator 48 having a multi-surface aerosol generator conductive portion 100. Figure 9a illustrates the aerosol generator 48 of figure 6b, where additionally the first, vaporisation surface conductive portion 110 is adjacent to a flow path from an air inlet 28 to an aerosol outlet 50, and air flow past the first, vaporisation surface conductive portion 110 is substantially parallel to the 2D planar extent (width or length) of the first, vaporisation surface conductive portion 110. The outlet 50 may be at least partially, if not fully (as shown), axially aligned with the air inlet 28, providing a substantially straight flow path from the inlet 28 to the outlet 50. Beneficially, this may further aid delivery of larger vapour droplets to the user.

In figure 9a, an angle θ between the first surface 71 and the second surface 72 is 90°, i.e. the first and second surfaces 71, 72 (and the respective first and second surface conductive portions 110, 120 thereon) are perpendicular, but more generally, in other examples, angle θ may be non-zero, i.e. the first and second surfaces 71, 72 (and the respective first and second surface conductive portions 110, 120 thereon) are not parallel. The flow path past the conductive portion 100 is as described above with reference to figure 8a, where the figure 9a system 1 additionally comprises two flow tubes 55a, 55b, with a first tube 55a for receiving air from the air inlet 28 and providing a conduit to the first surface conductive portion 110, where vapour is generated and entrained into the air flow to form aerosol, and a second tube 55b providing a conduit downstream of the first surface conductive portion 110 to the mouthpiece outlet 50. The tubes 55 provide a flow channel for the air/aerosol and may be configured to minimize (direct) exposure of other, non-vaporisation surfaces/portions (e.g. the second surface 72 and/or second surface conductive portion 120), and/or other components such as the conductive elements 60, as outlined further later, to the fluid flow, e.g. by being suitably located such as downstream of these surfaces and/or components. In figure 9a, the first tube 55a extends downstream from the air inlet 28 beyond both the second surface 72 and the second surface conductive portion 120, thus minimises exposure of these to the air flow.

Figure 9a also illustrates two reservoirs 44a, 44b of aerosol-generating material for supplying the aerosol generator 48, which here comprises a porous body and transports the aerosol-generating material to the vaporization surface 71 for aerosolisation at the first, vaporisation surface conductive portion 110. The first reservoir 44a is on a side wall of the aerosol generator 48 in the orientation shown (with the aerosol outlet 50 vertically above the air inlet 28) and aerosol-generating material is transported to the vaporization surface 71 by capillary action. The second reservoir 44b is vertically above the aerosol generator 48 in the orientation shown and thus flow from the second reservoir 44b to the aerosol generator 48 is aided by gravity in this orientation. The reservoir 44 is omitted in figures 9b, 9c and 10a, 10b for clarity and simplicity, but any one or more reservoirs 44 may be present in these examples also.

Figure 9b illustrates another system 1 comprising an aerosol generator 48 having a multi-surface aerosol generator conductive portion 100, but here the aerosol generator 48 has a rectangular shape with a cut / snipped bottom-left corner. In figure 9b, the multi-surface aerosol generator conductive portion 100 extends across first, second and third surfaces 71, 72, 73, where the angle θ between the first and second surfaces 71, 72 (and the first and second surface conductive portions 110, 120 thereon), measured relative to parallel, (i.e. for parallel surfaces, θ = 0°) is 45°. Additionally, the angle θ between the second and third surfaces 72, 73 (and between the second and third surface conductive portions 120, 130 thereon) is also 45°.

In figure 9b, the system 1 also comprises a tube 55 for directing air flow from the air inlet 28. In contrast to figure 9a, the tube 55 terminates upstream of the second and third surfaces 72, 73. Further, the first and second surface conductive portions 110, 120 extend into the air flow pathway from the tube 55, at least partially obstructing the linear, direct flow path from the inlet 28 to the outlet 50, thus are exposed to the fluid flow. The second surface conductive portion 120 may aid to channel/deflect flow to the outlet 50. The system 1 of figure 9b also comprises a cylindrical electrically conductive element 60 for connecting the conductive portion 100 to a power supply 26. The conductive element 60 contacts the horizontal third surface conductive portion 130 and has a length parallel to, but is spaced from, the direct flow path from the inlet 28 to the outlet 50 - in this example, the conductive element 60 is spaced from the tube 55 and has some (indirect) exposure to the flow path, but does not extend into it. Beneficially, in this orientation, the aerosol generator 48 is also supported in this orientation by the conductive element 60, and the conductive element 60 and conductive portion 100 (specifically the third surface conductive portion 130 thereof) make electrical contact at a horizontal interface, making reliable contact aided by gravity.

Figure 9c illustrates another system 1 comprising an aerosol generator 48 with a cut / snipped bottom-left corner. By contrast to figure 9b, in this example, the electrically conductive element 60 has a tapered tip, shaped at 45° to horizontal/vertical, to complement the second surface 72 comprising the second surface conductive portion 120, extending at 45° away from the first surface 71 comprising the first surface conductive portion 110. The conductive element 60 contacts the second surface conductive portion 120 and is substantially flush with a leading edge of the first surface conductive portion 110 towards the air flow path, thus the electrically conductive element 60 extends adjacent to the flow path from the inlet 28 to the outlet 50, having a length parallel to the flow path, but not protruding beyond the conductive portion 100 towards the flow path. More specifically, in some examples, the electrically conductive element 60 may not protrude beyond the first surface conductive portion 110 towards the air flow path, or particularly not beyond any higher resistance conductive portion, where present, which provides a primary vaporisation portion.

In figure 9c, a tube 55 receives air from the air inlet 28 and provides a conduit from the inlet 28 to the first surface conductive portion 110, where vapour is generated and entrained into the air flow to form aerosol, which flows to the mouthpiece outlet 50. The tube 55 provides a flow channel for air that opens downstream of both the conductive element 60 and the second surface 72, and so the electrically conductive element 60, second surface 72 and second surface conductive portion 120 do not extend or protrude into the (linear) flow path, nor are they directly exposed to the fluid flow.

Although specific examples where angle θ is 45° or 90° are outlined above and illustrated, angle θ may more generally be non-zero, or optionally lie substantially between 30-150°, 45-135° or particularly 45-90°. These arrangements provide a notable projection away from the first surface 71 and the first surface conductive portion 110 thereon that can be mirrored by a contact surface of the conductive element 60 to support the aerosol generator 48.

### Curved aerosol generator conductive portion

Figures 10a-10b are cross-section views of systems 1 comprising an aerosol generator 48 having a curved aerosol generator conductive portion 200, the curved conductive portion 200 having a curvature angle of substantially 30-150°, 45-135° or particularly 45-90°. These examples apply the same principles for planar surfaces above to curved surfaces, where e.g. tangents at each end of a substantially 90° curved conductive surface are substantially perpendicular, akin to perpendicular planar first and second surfaces 71, 72 comprising planar first and second surface conductive portions 110, 120 thereon. The conductive portion 200 itself may otherwise be as described above for single and multi-surface portions 49, 100. Flow tubes 55 and reservoirs 44 are omitted in the systems 1 of figures 10a-10b for clarity, but may still be provided, optionally e.g. in accordance with the figure 9a-9c examples.

Figure 10a illustrates a system 1 corresponding to figure 9a, but with a cylindrical aerosol generator 48 having a curved surface 75 and a curved conductive portion 200 on the curved surface 75. The curved conductive portion 200 has a curvature angle θ of 90° between first and second ends 210, 220 of the curved conductive portion 200, around the curved surface 75. The first end 210 of the curved conductive portion 200 is a leading edge, adjacent to an air flow path from an air inlet 28 to an outlet 50. Figure 10b illustrates another arrangement, here with a curvature angle θ of substantially 30° between the first and second ends 210, 220 of the curved conductive portion 200.

### Non-protruding conductive element

As outlined above, particularly with reference to figures 8a and 8b, this disclosure outlines aerosol delivery systems 1 comprising a conductive portion 49, 100, 200 that may extend across a single surface or across multiple surfaces of an aerosol generator 48, and outlines the benefits of a conductive element 60 that does not protrude beyond the conductive portion 49, 100, 200 towards an air flow path. In some examples, the conductive portion 49, 100, 200 may comprise a leading edge adjacent to or in the flow path, where the conductive element 60 does not protrude beyond the leading edge towards the flow path. Accordingly, this disclosure also encompasses an aerosol delivery system 1, or a cartridge for an aerosol delivery system 1, comprising: an aerosol generator 48 having an electrically conductive portion 49, 100, 200 for generating aerosol from aerosol-generating material in use; an electrically conductive element 60 for connecting the electrically conductive portion 49, 100, 200 to a power supply 26; and a flow path past the conductive portion 49, 100, 200 for entraining aerosol generated by the aerosol generator 48 into air from an air inlet 28, wherein the conductive portion 49, 100, 200 is exposed to the flow path and the conductive element 60 does not protrude beyond the conductive portion 49, 100, 200 towards the flow path.

Figure 11 is a cross-section view of a system 1 comprising an aerosol generator 48 having a conductive portion 49 and an electrically conductive element 60 contacting a bottom edge of the conductive portion 49, for connecting the conductive portion 49 to a power supply 26. The conductive element 60 is vertically aligned with the planar extent of the conductive portion 49.

The system 1 also comprises a flow path from an air inlet 28 to the conductive portion 49, for entraining aerosol generated by the aerosol generator 48 into air from the air inlet 28, for transport to an aerosol outlet 50.

The conductive portion 49 is exposed to the flow path and the conductive element 60 does not protrude beyond the conductive portion 49 towards the flow path, i.e. the conductive element 60 is substantially flush with the conductive portion 49. The system 1 also comprises a flow tube 55 providing a conduit for air from the air inlet 28 to the conductive portion 49, where here the tube 55 extends from the inlet 28 downstream beyond the conductive element 60, minimising exposure of the conductive element 60 to the fluid flow, as in the figure 9c example, where the conductive element 60 is not directly exposed to the air flow path.

In one further example (not shown), the conductive portion 49 comprises a mesh extending / protruding beyond the first surface 71 of the aerosol generator 48 to provide a connecting part parallel to the first surface 71, where the conductive element 60 contacts the connecting part, but does not protrude beyond the conductive portion 49 towards the flow path.

For the avoidance of any doubt, these example are envisaged in any combination with other features set out above. Equally, the ordinal reference labels (first, second, third etc.) are arbitrary and simply for convenience.

The various embodiments described herein are presented only to assist in understanding and teaching the claimed features. These embodiments are provided as a representative sample of embodiments only, and are not exhaustive and/or exclusive. Any functions of a processor (e.g. controller) may be shared between processors on the various devices/systems in the wider system and/or a remote server. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention.

Various embodiments of the invention may suitably comprise, consist of, or consist essentially of, appropriate combinations of the disclosed elements, components, features, parts, steps, means, etc., other than those specifically described herein. In addition, this disclosure may include other inventions not presently claimed, but which may be claimed in future. Protection may also be sought for any features disclosed in any one or more published documents referenced herein in combination with the present disclosure.

### Terminology

### Delivery System

As used herein, the term "delivery system" is intended to encompass systems that deliver at least one substance to a user in use, and includes:
combustible aerosol provision systems, such as cigarettes, cigarillos, cigars, and tobacco for pipes or for roll-your-own or for make-your-own cigarettes (whether based on tobacco, tobacco derivatives, expanded tobacco, reconstituted tobacco, tobacco substitutes or other smokable material);
non-combustible aerosol provision systems that release compounds from an aerosol-generating material without combusting the aerosol-generating material, such as electronic cigarettes, tobacco heating products, and hybrid systems to generate aerosol using a combination of aerosol-generating materials; and
aerosol-free delivery systems that deliver the at least one substance to a user orally, nasally, transdermally or in another way without forming an aerosol, including but not limited to, lozenges, gums, patches, articles comprising inhalable powders, and oral products such as oral tobacco which includes snus or moist snuff, wherein the at least one substance may or may not comprise nicotine.

### Combustible Aerosol Provision System

According to the present disclosure, a "combustible" aerosol provision system is one where a constituent aerosol-generating material of the aerosol provision system (or component thereof) is combusted or burned during use in order to facilitate delivery of at least one substance to a user.

In some embodiments, the delivery system is a combustible aerosol provision system, such as a system selected from the group consisting of a cigarette, a cigarillo and a cigar. In some embodiments, the disclosure relates to a component for use in a combustible aerosol provision system, such as a filter, a filter rod, a filter segment, a tobacco rod, a spill, an aerosol-modifying agent release component such as a capsule, a thread, or a bead, or a paper such as a plug wrap, a tipping paper or a cigarette paper.

### Non-Combustible Aerosol Provision System

According to the present disclosure, a "non-combustible" aerosol provision system is one where a constituent aerosol-generating material of the aerosol provision system (or component thereof) is not combusted or burned in order to facilitate delivery of at least one substance to a user.

In some embodiments, the delivery system is a non-combustible aerosol provision system, such as a powered non-combustible aerosol provision system. In some embodiments, the non-combustible aerosol provision system is an electronic cigarette, also known as a vaping device or electronic nicotine delivery system (END), although it is noted that the presence of nicotine in the aerosol-generating material is not a requirement. In some embodiments, the non-combustible aerosol provision system is an aerosol-generating material heating system, also known as a heat-not-burn system. An example of such a system is a tobacco heating system.

In some embodiments, the non-combustible aerosol provision system is a hybrid system to generate aerosol using a combination of aerosol-generating materials, one or a plurality of which may be heated. Each of the aerosol-generating materials may be, for example, in the form of a solid, liquid or gel and may or may not contain nicotine. In some embodiments, the hybrid system comprises a liquid or gel aerosol-generating material and a solid aerosol-generating material. The solid aerosol-generating material may comprise, for example, tobacco or a non-tobacco product.

Typically, the non-combustible aerosol provision system may comprise a non-combustible aerosol provision device and a consumable for use with the non-combustible aerosol provision device. In some embodiments, the disclosure relates to consumables comprising aerosol-generating material and configured to be used with non-combustible aerosol provision devices. These consumables are sometimes referred to as articles throughout the disclosure.

In some embodiments, the non-combustible aerosol provision system, such as a non-combustible aerosol provision device thereof, may comprise a power source and a controller. The power source may, for example, be an electric power source or an exothermic power source. In some embodiments, the exothermic power source comprises a carbon substrate which may be energised so as to distribute power in the form of heat to an aerosol-generating material or to a heat transfer material in proximity to the exothermic power source.

In some embodiments, the non-combustible aerosol provision system may comprise an area for receiving the consumable, an aerosol generator, an aerosol generation area, a housing, a mouthpiece, a filter and/or an aerosol-modifying agent. In some embodiments, the consumable for use with the non-combustible aerosol provision device may comprise aerosol-generating material, an aerosol-generating material storage area, an aerosol-generating material transfer component, an aerosol generator, an aerosol generation area, a housing, a wrapper, a filter, a mouthpiece, and/or an aerosol-modifying agent.

### Aerosol-Free Delivery System

In some embodiments, the delivery system is an aerosol-free delivery system that delivers at least one substance to a user orally, nasally, transdermally or in another way without forming an aerosol, including but not limited to, lozenges, gums, patches, articles comprising inhalable powders, and oral products such as oral tobacco which includes snus or moist snuff, wherein the at least one substance may or may not comprise nicotine.

In some embodiments, the substance to be delivered may be an aerosol-generating material or a material that is not intended to be aerosolised. As appropriate, either material may comprise one or more active constituents, one or more flavours, one or more aerosol-former materials, and/or one or more other functional materials.

### Active Substance

In some embodiments, the substance to be delivered comprises an active substance. The active substance as used herein may be a physiologically active material, which is a material intended to achieve or enhance a physiological response. The active substance may for example be selected from nutraceuticals, nootropics, psychoactives. The active substance may be naturally occurring or synthetically obtained. The active substance may comprise for example nicotine, caffeine, taurine, theine, vitamins such as B6 or B12 or C, melatonin, cannabinoids, or constituents, derivatives, or combinations thereof. The active substance may comprise one or more constituents, derivatives or extracts of tobacco, cannabis or another botanical. In one embodiment the active substance is a legally permissible recreational drug. In some embodiments, the active substance comprises nicotine. In some embodiments, the active substance comprises caffeine, melatonin or vitamin B12.

As noted herein, the active substance may comprise one or more constituents, derivatives or extracts of cannabis, such as one or more cannabinoids or terpenes. The active substance may be CBD or a derivative thereof. As noted herein, the active substance may comprise or be derived from one or more botanicals or constituents, derivatives or extracts thereof. As used herein, the term "botanical" includes any material derived from plants including, but not limited to, extracts, leaves, bark, fibres, stems, roots, seeds, flowers, fruits, pollen, husk, shells or the like. Alternatively, the material may comprise an active compound naturally existing in a botanical, obtained synthetically. The material may be in the form of liquid, gas, solid, powder, dust, crushed particles, granules, pellets, shreds, strips, sheets, or the like.

Example botanicals are tobacco, eucalyptus, star anise, hemp, cocoa, cannabis, fennel, lemongrass, peppermint, spearmint, rooibos, chamomile, flax, ginger, ginkgo biloba, hazel, hibiscus, laurel, licorice (liquorice), matcha, mate, orange skin, papaya, rose, sage, tea such as green tea or black tea, thyme, clove, cinnamon, coffee, aniseed (anise), basil, bay leaves, cardamom, coriander, cumin, nutmeg, oregano, paprika, rosemary, saffron, lavender, lemon peel, mint, juniper, elderflower, vanilla, wintergreen, beefsteak plant, curcuma, turmeric, sandalwood, cilantro, bergamot, orange blossom, myrtle, cassis, valerian, pimento, mace, damien, marjoram, olive, lemon balm, lemon basil, chive, carvi, verbena, tarragon, geranium, mulberry, ginseng, theanine, theacrine, maca, ashwagandha, damiana, guarana, chlorophyll, baobab or any combination thereof. The mint may be chosen from the following mint varieties: Mentha Arventis, Mentha c.v.,Mentha niliaca, Mentha piperita, Mentha piperita citrata c.v.,Mentha piperita c.v, Mentha spicata crispa, Mentha cardifolia, Memtha longifolia, Mentha suaveolens variegata, Mentha pulegium, Mentha spicata c.v. and Mentha suaveolens.

In some embodiments, the active substance comprises or is derived from one or more botanicals or constituents, derivatives or extracts thereof and the botanical is tobacco. In some embodiments, the active substance comprises or derived from one or more botanicals or constituents, derivatives or extracts thereof and the botanical is selected from eucalyptus, star anise, cocoa and hemp. In some embodiments, the active substance comprises or derived from one or more botanicals or constituents, derivatives or extracts thereof and the botanical is selected from rooibos and fennel.

### Flavours

In some embodiments, the substance to be delivered comprises a flavour. As used herein, the terms "flavour" and "flavourant" refer to materials which, where local regulations permit, may be used to create a desired taste, aroma or other somatosensorial sensation in a product for adult consumers. They may include naturally occurring flavour materials, botanicals, extracts of botanicals, synthetically obtained materials, or combinations thereof (e.g., tobacco, cannabis, licorice (liquorice), hydrangea, eugenol, Japanese white bark magnolia leaf, chamomile, fenugreek, clove, maple, matcha, menthol, Japanese mint, aniseed (anise), cinnamon, turmeric, Indian spices, Asian spices, herb, wintergreen, cherry, berry, red berry, cranberry, peach, apple, orange, mango, clementine, lemon, lime, tropical fruit, papaya, rhubarb, grape, durian, dragon fruit, cucumber, blueberry, mulberry, citrus fruits, Drambuie, bourbon, scotch, whiskey, gin, tequila, rum, spearmint, peppermint, lavender, aloe vera, cardamom, celery, cascarilla, nutmeg, sandalwood, bergamot, geranium, khat, naswar, betel, shisha, pine, honey essence, rose oil, vanilla, lemon oil, orange oil, orange blossom, cherry blossom, cassia, caraway, cognac, jasmine, ylang-ylang, sage, fennel, wasabi, piment, ginger, coriander, coffee, hemp, a mint oil from any species of the genus Mentha, eucalyptus, star anise, cocoa, lemongrass, rooibos, flax, ginkgo biloba, hazel, hibiscus, laurel, mate, orange skin, rose, tea such as green tea or black tea, thyme, juniper, elderflower, basil, bay leaves, cumin, oregano, paprika, rosemary, saffron, lemon peel, mint, beefsteak plant, curcuma, cilantro, myrtle, cassis, valerian, pimento, mace, damien, marjoram, olive, lemon balm, lemon basil, chive, carvi, verbena, tarragon, limonene, thymol, camphene), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, liquid such as an oil, solid such as a powder, or gas.

In some embodiments, the flavour comprises menthol, spearmint and/or peppermint. In some embodiments, the flavour comprises flavour components of cucumber, blueberry, citrus fruits and/or redberry. In some embodiments, the flavour comprises eugenol. In some embodiments, the flavour comprises flavour components extracted from tobacco. In some embodiments, the flavour comprises flavour components extracted from cannabis.

In some embodiments, the flavour may comprise a sensate, which is intended to achieve a somatosensorial sensation which are usually chemically induced and perceived by the stimulation of the fifth cranial nerve (trigeminal nerve), in addition to or in place of aroma or taste nerves, and these may include agents providing heating, cooling, tingling, numbing effect. A suitable heat effect agent may be, but is not limited to, vanillyl ethyl ether and a suitable cooling agent may be, but not limited to eucolyptol, WS-3.

### Aerosol-generating material

Aerosol-generating material is a material that is capable of generating aerosol, for example when heated, irradiated or energized in any other way. Aerosol-generating material may, for example, be in the form of a solid, liquid or semi-solid (such as a gel) which may or may not contain an active substance and/or flavourants. The aerosol-generating material may comprise one or more active substances and/or flavours, one or more aerosol-former materials, and optionally one or more other functional material.

The aerosol-generating material may comprise a binder, such as a gelling agent, and an aerosol former. Optionally, a substance to be delivered and/or filler may also be present. Optionally, a solvent, such as water, is also present and one or more other components of the aerosol-generating material may or may not be soluble in the solvent. In some embodiments, the aerosol-generating material is substantially free from botanical material. In particular, in some embodiments, the aerosol-generating material is substantially tobacco free.

The aerosol-generating material may comprise or be in the form of an aerosol-generating film. The aerosol-generating film may comprise a binder, such as a gelling agent, and an aerosol former. Optionally, a substance to be delivered and/or filler may also be present. The aerosol-generating film may be substantially free from botanical material. In particular, in some embodiments, the aerosol-generating material is substantially tobacco free. The aerosol-generating film may have a thickness of about 0.015 mm to about 1 mm. For example, the thickness may be in the range of about 0.05 mm, 0.1 mm or 0.15 mm to about 0.5 mm or 0.3 mm. The aerosol-generating material may comprise more than one film, and the thickness described herein may refer to the aggregate thickness of those films.

The aerosol-generating film may be continuous. For example, the film may comprise or be a continuous sheet of material. The sheet may be in the form of a wrapper, it may be gathered to form a gathered sheet or it may be shredded to form a shredded sheet. The shredded sheet may comprise one or more strands or strips of aerosol-generating material. The aerosol-generating film may be discontinuous. For example, the aerosol-generating film may comprise one or more discrete portions or regions of aerosol-generating material, such as dots, stripes or lines, which may be supported on a support. In such embodiments, the support may be planar or non-planar.

The aerosol-generating film may be formed by combining a binder, such as a gelling agent, with a solvent, such as water, an aerosol-former and one or more other components, such as one or more substances to be delivered, to form a slurry and then heating the slurry to volatilise at least some of the solvent to form the aerosol-generating film. The slurry may be heated to remove at least about 60 wt%, 70 wt%, 80 wt%, 85 wt% or 90 wt% of the solvent.

The aerosol-generating material may comprise or be an "amorphous solid". In some embodiments, the aerosol-generating materiel comprises an aerosol-generating film that is an amorphous solid. The amorphous solid may be a "monolithic solid". The amorphous solid may be substantially non-fibrous. In some embodiments, the amorphous solid may be a dried gel. The amorphous solid is a solid material that may retain some fluid, such as liquid, within it. In some embodiments, the amorphous solid may, for example, comprise from about 50wt%, 60wt% or 70wt% of amorphous solid, to about 90wt%, 95wt% or 100wt% of amorphous solid.

The amorphous solid may be substantially free from botanical material. The amorphous solid may be substantially tobacco free.

### Aerosol-former material

The aerosol-former material may comprise one or more constituents capable of forming an aerosol. In some embodiments, the aerosol-former material may comprise one or more of glycerol, propylene glycol, diethylene glycol, triethylene glycol, tetraethylene glycol, 1 ,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate.

### Functional material

The one or more other functional materials may comprise one or more of pH regulators, colouring agents, preservatives, binders, fillers, stabilizers, and/or antioxidants.

### Substrate

The material may be present on or in a support, to form a substrate. The support may, for example, be or comprise paper, card, paperboard, cardboard, reconstituted material, a plastics material, a ceramic material, a composite material, glass, a metal, or a metal alloy. In some embodiments, the support comprises a susceptor. In some embodiments, the susceptor is embedded within the material. In some alternative embodiments, the susceptor is on one or either side of the material.

### Consumable

A consumable is an article comprising or consisting of aerosol-generating material, part or all of which is intended to be consumed during use by a user. A consumable may comprise one or more other components, such as an aerosol-generating material storage area, an aerosol-generating material transfer component, an aerosol generation area, a housing, a wrapper, a mouthpiece, a filter and/or an aerosol-modifying agent. A consumable may also comprise an aerosol generator, such as a heater, that emits heat to cause the aerosol-generating material to generate aerosol in use. The heater may, for example, comprise combustible material, a material heatable by electrical conduction, or a susceptor.

### Susceptor

A susceptor is a material that is heatable by penetration with a varying magnetic field, such as an alternating magnetic field. The susceptor may be an electrically-conductive material, so that penetration thereof with a varying magnetic field causes induction heating of the heating material. The heating material may be magnetic material, so that penetration thereof with a varying magnetic field causes magnetic hysteresis heating of the heating material. The susceptor may be both electrically-conductive and magnetic, so that the susceptor is heatable by both heating mechanisms. The device that is configured to generate the varying magnetic field is referred to as a magnetic field generator, herein.

### Aerosol-modifying agent

An aerosol-modifying agent is a substance, typically located downstream of the aerosol generation area, that is configured to modify the aerosol generated, for example by changing the taste, flavour, acidity or another characteristic of the aerosol. The aerosol-modifying agent may be provided in an aerosol-modifying agent release component, that is operable to selectively release the aerosol-modifying agent. The aerosol-modifying agent may, for example, be an additive or a sorbent. The aerosol-modifying agent may, for example, comprise one or more of a flavourant, a colourant, water, and a carbon adsorbent. The aerosol-modifying agent may, for example, be a solid, a liquid, or a gel. The aerosol-modifying agent may be in powder, thread or granule form. The aerosol-modifying agent may be free from filtration material.

### Aerosol generator

An aerosol generator is an apparatus configured to cause aerosol to be generated from the aerosol-generating material. In some embodiments, the aerosol generator is a heater configured to subject the aerosol-generating material to heat energy, so as to release one or more volatiles from the aerosol-generating material to form an aerosol. In some embodiments, the aerosol generator is configured to cause an aerosol to be generated from the aerosol-generating material without heating. For example, the aerosol generator may be configured to subject the aerosol-generating material to one or more of vibration, increased pressure, or electrostatic energy.

The present disclosure relates to aerosol delivery systems (which may also be referred to as vapour delivery systems) such as nebulisers or e-cigarettes. Throughout the following description the term "e-cigarette" or "electronic cigarette" may sometimes be used, but it will be appreciated this term may be used interchangeably with aerosol delivery system / device and electronic aerosol delivery system / device. Furthermore, and as is common in the technical field, the terms "aerosol" and "vapour", and related terms such as "vaporise", "volatilise" and "aerosolise", may generally be used interchangeably.

Aerosol delivery systems (e-cigarettes) often, though not always, comprise a modular assembly comprising a reusable device part and a replaceable (disposable/consumable) cartridge part. Often, the replaceable cartridge part will comprise the aerosol generating material and the vaporiser (which may collectively be called a 'cartomizer') and the reusable device part will comprise the power supply (e.g. rechargeable power source) and control circuitry. It will be appreciated these different parts may comprise further elements depending on functionality. For example, the reusable device part will often comprise a user interface for receiving user input and displaying operating status characteristics, and the replaceable cartridge device part in some cases comprises a temperature sensor for helping to control temperature. Cartridges are electrically and mechanically coupled to the control unit for use, for example using a screw thread, bayonet, or magnetic coupling with appropriately arranged electrical contacts. When the aerosol generating material in a cartridge is exhausted, or the user wishes to switch to a different cartridge having a different aerosol generating material, the cartridge may be removed from the reusable part and a replacement cartridge attached in its place. Systems and devices conforming to this type of two-part modular configuration may generally be referred to as two-part systems/devices.

It is common for electronic cigarettes to have a generally elongate shape. For the sake of providing a concrete example, certain embodiments of the disclosure will be taken to comprise this kind of generally elongate two-part system employing disposable cartridges. However, it will be appreciated that the underlying principles described herein may equally be adopted for different configurations, for example single-part systems or modular systems comprising more than two parts, refillable devices and single-use disposables, as well as other overall shapes, for example based on so-called box-mod high performance devices that typically have a boxier shape. More generally, it will be appreciated certain embodiments of the disclosure are based on aerosol delivery systems which are operationally configured to provide functionality in accordance with the principles described herein and the constructional aspects of systems configured to provide the functionality in accordance with certain embodiments of the disclosure is not of primary significance.

Throughout the disclosure, the terms 'substantially', 'approximately' and 'about' should be considered to mean within +/- 10% unless indicated otherwise.

### Index to reference numerals

- 1: aerosol delivery system
- 2: reusable part
- 4: cartridge part
- 6: interface between reusable part and cartridge part
- 12: reusable part housing
- 14, 16: user input buttons
- 20: user programming circuitry
- 22: controller
- 24: display
- 26: power source
- 28: air inlet
- 30: airflow sensor
- 31: printed circuit board (PCB)
- 32: sensor cavity or chamber
- 34: chamber wall
- 42: cartridge housing
- 44: chamber or reservoir
- 46: wick
- 48: aerosol generator
- 49: aerosol generator conductive portion
- 50: mouthpiece outlet
- 51: airflow path through reusable part
- 52: airflow path through cartridge
- 55: tube
- 60: conductive element
- 71: aerosol generator first surface
- 72: aerosol generator second surface
- 73: aerosol generator third surface
- 75: aerosol generator curved surface
- 100: multi-surface aerosol generator conductive portion
- 110: first surface conductive portion
- 112: first surface inner conductive portion
- 114: first surface outer conductive portion
- 120: second surface conductive portion
- 130: third surface conductive portion
- 150: internal conductive portion
- 200: curved aerosol generator conductive portion
- 210: first end of curved aerosol generator conductive portion
- 220: second end of curved aerosol generator conductive portion

## Claims

1. An aerosol generator for an aerosol delivery system, the aerosol generator having an electrically conductive portion for generating aerosol from aerosol-generating material in use, wherein the conductive portion extends from a first surface of the aerosol generator to a second, different surface of the aerosol generator.

2. The aerosol generator of claim 1, wherein an angle between the first surface and the second surface is substantially 30-150°, 45-135° or 45-90°.

3. An aerosol generator for an aerosol delivery system, the aerosol generator having an electrically conductive portion for generating aerosol from aerosol-generating material in use, wherein the conductive portion comprises a curved surface having a curvature angle of substantially 30-150°, 45-135° or 45-90°.

4. An aerosol delivery system or a cartridge for an aerosol delivery system, the system or cartridge comprising the aerosol generator of any of claims 1-3, optionally further comprising aerosol-generating material.

5. The aerosol generator, system or cartridge of any preceding claim, wherein the aerosol generator comprises a porous body, configured to transport aerosol-generating material to the conductive portion for aerosolisation.

6. The system or cartridge of any preceding claim, comprising a reservoir of aerosol-generating material configured to deliver aerosol-generating material to a surface of the aerosol generator that substantially opposes the first or the second surface of the aerosol generator.

7. The aerosol generator, system or cartridge of any preceding claim, wherein the conductive portion comprises:
a. a higher resistance portion, for generating aerosol from aerosol-generating material by resistance heating; and
b. a lower resistance portion, for supplying power to the higher resistance portion.

8. The aerosol generator, system or cartridge of any preceding claim, wherein the conductive portion comprises on the first surface:
a. an inner, higher resistance portion, for generating aerosol from aerosol-generating material by resistance heating; and
b. a pair of outer, lower resistance portions, one either side of the inner, higher resistance portion, for supplying power thereto.

9. The aerosol generator, system or cartridge of claim 7 or 8, wherein the lower resistance portion(s) extend from the first surface of the aerosol generator to the second surface of the aerosol generator.

10. The system or cartridge of any of claims 4 to 9, further comprising an electrically conductive element for connecting the conductive portion to a power supply.

11. The system or cartridge of claim 10, wherein the conductive portion is exposed to an air flow path from an air inlet to an aerosol outlet and the electrically conductive element does not protrude into the air flow path.

12. The system or cartridge of claim 10, wherein the electrically conductive element has an axis of extent along a length that is substantially parallel to an axis of extent along a length of an airflow path from an air inlet to an aerosol outlet.

13. The system or cartridge of any of claims 4 to 12, wherein the first surface comprises a vaporisation surface that is:
a. adjacent to an airflow path from an air inlet to an aerosol outlet; and/or
b. substantially parallel to an air flow path past the vaporisation surface.

14. The system or cartridge of any of claims 4 to 13, comprising an aerosol outlet at least partially axially aligned with an air inlet, providing a substantially straight flow path from the inlet to the outlet.

15. An aerosol delivery system comprising the aerosol generator of any preceding claim, wherein:
a. the aerosol generator comprises a porous body, configured to transport aerosol-generating material to the conductive portion for aerosolisation, wherein the first and second surfaces are planar and substantially perpendicular to one another;
b. the conductive portion comprises:
i. a higher resistance portion configured to generate aerosol from aerosol-generating material by resistance heating; and
ii. a lower resistance portion, configured to supply power to the higher resistance portion; and
c. the system comprises an air inlet and an aerosol outlet, wherein the aerosol outlet is at least partially axially aligned with the air inlet, providing a substantially straight flow path from the air inlet, past the higher resistance portion, to the aerosol outlet, wherein the flow path is substantially parallel to the width or length of the higher resistance conductive portion.
